# EUROPEAN PATENT SPECIFICATION

(11) **EP 1 367 052 B1**
(45) Date of publication and mention of the grant of the patent: **12.07.2006**
(21) Application number: 03077684.3
(22) Date of filing: 03.05.2001
(51) Int. Cl.: C07D 209/88, C07D 263/20, C07C 271/16, C07C 69/96

(54) **Intermediate for the preparation of carvedilol**
Zwischenverbindung für die Herstellung von Carvedilol
Intermédiaire pour la préparation de carvedilol

(30) Priority: 18.05.2000 DK 200000801
(43) Date of publication of application: 03.12.2003
(62) Divisional of application: 01929337.2
(73) Proprietor: A/S GEA Farmaceutisk Fabrik, 2650 Hvidovre (DK)
(72) Inventor: Pittelkow, Thomas, DK-4520 Svinninge (DK); Fischer, Erik, DK-3500 Vaerlose (DK); Treppendahl, Svend Peter, 2830 Virum (DK)
(74) Representative: Rotne, Jens Styrup

(56) References cited:
- US-A- 3 152 168

## Description

### FIELD OF INVENTION

The present invention relates to new intermediates having the formula (V), 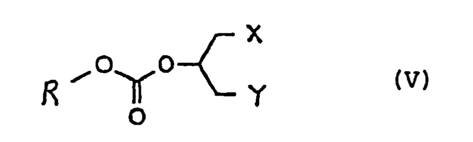 wherein X and Y each is a leaving group as defined below, and R is seleted from phenyl and phenyl substituted with one or more electrophilic substituents as defined below.

The compound having the formula (V) is useful in the preparation of carvedilol, 1-(9H-carbazol-4-yloxy)-3-[2- (2-methoxy-phenoxy)-ethylamino]-propan-2-ol. This compound is a valuable pharmaceutical substance, which is used as a medicament having antihypertensive beta-adrenergic blocking and vasodilating activity. It is usually termed carvedilol and exists in at least two solid forms having differing physio-chemical properties. The conversion from one form to another is described in EP 0 893 440.

Further the invention relates to the preparation of said new intermediates and use thereof for the preparation of carvedilol.

### BACKGROUND FOR THE INVENTION

Various routs of synthesis have been used or suggested for the preparation of carvedilol.

Thus EP 0 004 920 described reaction of 4-(oxiranylmethoxy)-9H-carbazol with 2-(2-methoxy-phenoxy)-ethylamine. 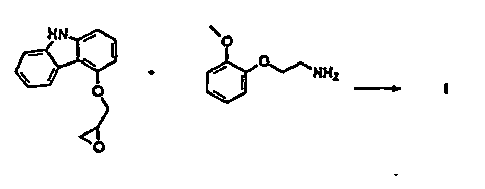 In this process also the bis compound (IV) is formed.

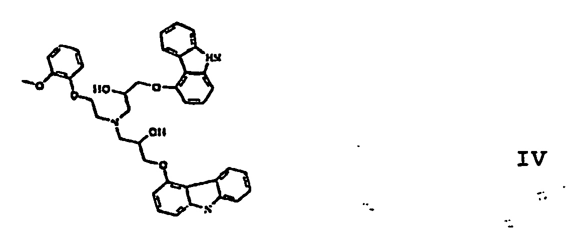

The starting compound 4-(oxiranylmethoxy)-9H-carbazol can be prepared by reaction of 4-hydroxycarbazol with an oxiranyl derivative, usually in a low yield. This means that a large amount of the expensive compound 4-hydroxycarbazol is lost during the reactions for which reason the process is less favourable in economical respect. In addition the synthesis makes use of an oxiranyl derivative which is not desired for environmental reasons.

Further EP 0 004 920 suggests three additional strategies for formation of carvedilol where 4-[3'-amino-2'-(hydroxy)-propoxy]-9H-carbazole is reacted with:
1. 2-[2'-methoxy-phenoxy]-ethylhalide or sulphonate,
2. 2-[2'-methoxy-phenoxy]-acetaldehyde followed by catalytic hydrogenation, or
3. 2-[2'-methoxy-phenoxy]-acetylchloride followed by reduction of the formed amide using a complex metal hydride.

Neither of these strategies are attractive from an industrial, economical point of view because the processes involve separation of the formed product from the starting materials using column chromatography and because the starting materials are relatively expensive.

In EP 0 127 099 the preparation of the optically active R and S enantiomers of carvedilol is described, using an R or an S enantiomer of the oxiran-derivative as starting material for the synthesis. Further it is described that the two enantiomers have differing biological activities. Thus the S enantiomer has a beta-blocking activity whereas both the R and the S enantiomer have a vasodilating activity.

EP 0 918 055 describes that the formation of the bis side product (IV) can be avoided by mono protecting the N atom in 2-(2'-(methoxy)-phenoxy)-ethylamine before reaction with 4-(oxiranylmethoxy)-9H-carbazol using a benzyl group as protection group. The formed product benzyl-carvedilol has to be debenzylated before the desired product carvedilol is formed, which debenzylation is done by hydrogenative debenzylation. Thus this procedure introduces two additional steps in the synthesis, a benzylation and a debenzylation step.

In Maier, N.M. and Uray, G. (1996) J. Chromatogr. A, 740:11-19) the formation of 5-(9H-carbazol-4-yloxymethyl)-3-[2-(2-methoxy-phenoxy)-ethyl]-oxazolidin-2-one (IX) (the structure is shown below) is described by cyclisation of carvedilol with phosgene. The compound (IX) was used in a study of a particular stationary phase for use in chromatographic separations. Since according to this paper (IX) is made using carvedilol as starting material, the paper in no way inspires a person seeking new synthesis routes to carvedilol to consider (IX) as a starting material or an intermediate. The possibility of converting (IX) to carvedilol was not considered.

Thus there is a need for a new process for the synthesis of carvedilol not involving risks, hazards and high costs inherent in the above discussed prior art processes.

### BRIEF DESCRIPTION OF THE INVENTION

In one aspect the present invention provides new intermediates useful in a process for the manufacture of carvedilol, which process is economically and industrially feasible, which process does not make use of any hazardous oxiranyl derivatives.

The invention utilises the recognition that in a multi step synthesis it is advantageous to introduce the most expensive compound late in the synthesis in order to minimize the losses of this compound, by minimizing the number of steps after its introduction.

Thus in one aspect the invention relates to a process for the manufacture of 1-[9H-carbazol-4-yloxy]-3-[2-(2-methoxy-phenoxy)-ethylamino]-propan-2-ol, carvedilol, of the formula (I), or an acid addition salt thereof, 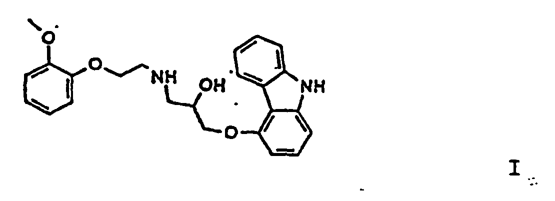 wherein a compound of the general formula (VII) 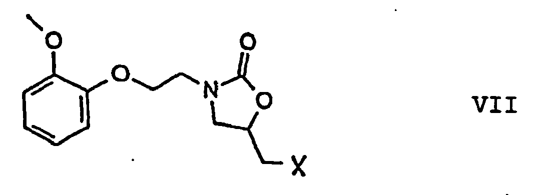 wherein X is a leaving group, where the leaving group X may be selected from, halogens (fluorine, chlorine, bromine and iodine), besylates, tosylate, mesylate, brosylates, nosylates, triflates, nonaflates or tresylates;
is alkylated with 4-hydroxycarbazol of the formula (VIII), 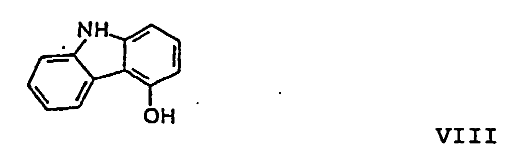 and the resulting compound 5-(9H-carbazol-4-yloxymethyl)-3-[2-(2-methoxy-phenoxy)-ethyl]-oxazolidin-2-one (IX) or an acid addition salt thereof 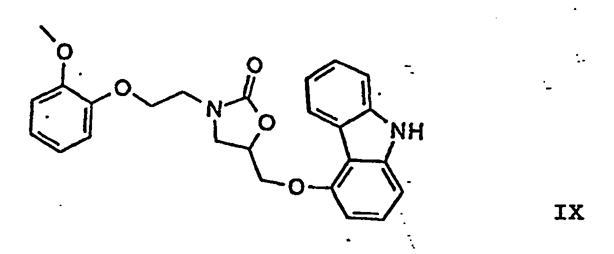 is hydrolysed.

This synthesis performs well in industrial scale with a yield of 50-60% (53% where X=chorine) based on the initial amount of the expensive compound 4-hydroxycarbazol.

Further the reaction proceeds without the use of any oxirane derivatives, which is desirable for environmental reasons and for the safety of the staff.

From the Japanese patent application Sho 62-108870 3-alkyl-5-hydroxy-oxazolidin-2-one derivates are known as precursors for the preparation of *β-*blockers or a,β-blockers. However the compound having the general formula (VII) can not be produced by the disclosure in said patent application, nor can any hint for the synthesis of this compound be deduced from said application.

In one embodiment the alkylation of the compound having the general formula (VII) with 4-hydroxycarbazol and the hydrolysis of the resulting compound having the general formula (IX) to 1-(9H-carbazol-4-yloxy)-3-[2-(2-methoxy-phenoxy)-ethylamino]-propan-2-ol is performed without purification of the intermediate compound having the general formula (IX). Consequently these two reactions may be performed in one container without the need for handling of the reaction product of the first reaction thereby avoiding the loss that inevitably occurs during handling of such a compound.

The compound having the general formula (VII) may be produced using several approaches.

In a preferred way of producing the compound of the general formula (VII) the oxazolidine ring of the compound is formed by cyclisation of a linear precursor having the general formula (VI) 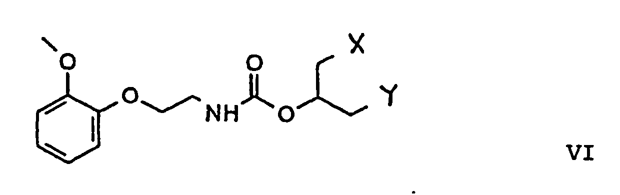 wherein X and Y each is a leaving group, where the leaving groups X and Y independently may be selected from halogens (fluorine, chlorine, bromine and iodine), besylates, tosylate, mesylate, brosylates, nosylates, triflates, nonaflates or tresylates.

The compound of the general formula (VI) may be formed by condensation of 2-(2-methoxy-phenoxy)-ethylamine and the compound having the general formula (V) 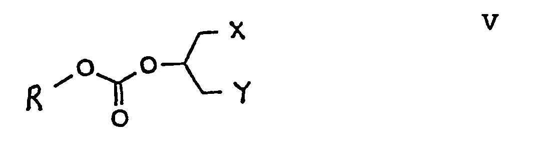 wherein X and Y have the above meaning and
R is selected so that R-O is a better leaving group than 1-X-3-Y-propan-2-oxy, as will be explained further below.

The compound having the general formula (V) may be formed by the following reaction: 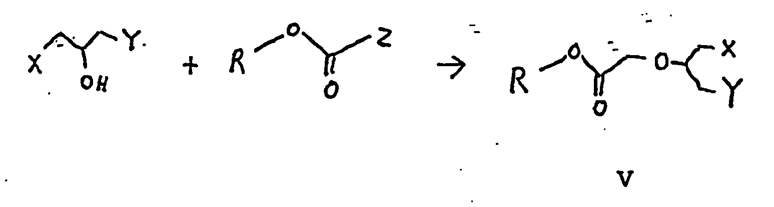 wherein X and Y each is a leaving group, where the leaving groups X and Y independently may be selected halogens (fluorine, chlorine, bromine and iodine), besylates, tosylate, mesylate, brosylates, nosylates, triflates, nonaflates or tresylates;
Z is a leaving group that may be selected from halogens (fluorine, chorine, bromine and iodine);
and R has the above meaning.

Thus the use of the compound having the formula (V) for the preparation of the compound having the formula (VI) forms a preferred aspect of the invention.

Another preferred aspect of the invention is the use of the compound having the formula (V) for the preparation of the compound having the formula (VI), a useful intermediate in the preparation of carvedilol.

A further preferred aspect of the invention is the use of the compound having the formula (V) for the preparation of the compound having the formula (VII), a useful intermediate in the preparation of carvedilol.

Further the use of the compound having the formula (V) for the preparation of carvedilol forms another preferred aspect of the invention.

Thus the invention provides new valuable intermediates for the synthesis of carvedilol and procedures for their production are provided.

### DETAILED DESCRIPTION OF THE INVENTION

The compound according to the invention having the general formula (V) may be prepared by the reaction of 1-X-3-Y-propan-2-ol and R-Z-formate as shown above.

The term leaving group is to be understood in the usual way, as it will be appreciated by the person skilled in the art. The selection of R is explained in more details further below.

For the purpose of this invention the groups X, Y and Z are leaving groups and they may be the same or different, as the leaving groups are lost during the reactions to follow and none of them will be present in the final product, carvedilol. The term "leaving group" is to be understood in the usual meaning as it will be known to the person skilled in the art. Thus the groups X, Y and Z are selected so that the reactions in which compounds containing said groups participate can be performed at a suitable rate. The groups X and Y are independently selected from: halogens (fluorine, chlorine, bromine and iodine), besylates, tosylate, mesylate, brosylates, nosylates, triflates, nonaflates, or thresylates. Z is selected from: halogens (fluorine, chlorine, bromine and iodine). Chlorine is an example of a preferred group X,Y and Z. The person skilled in the art will appreciate that other leaving groups than the above mentioned, known within the art may be used for the present invention.

The formation of the compound having the general formula (V) may be performed in an inert organic solvent in the presence of a base.

The solvent may in principle be any solvent that is inert under the conditions employed. Toluene is an example of an suitable solvent.

The base may in principle be any base. It is preferred that the base is soluble in the solvent. Triethylamine is an example of a preferred base for the reaction.

In order to prepare carvedilol the compound of the general formula (V) may be reacted with 2-(2-(methoxy-phenoxy)-ethylamine (III) to form the compound of the general formula (VI).

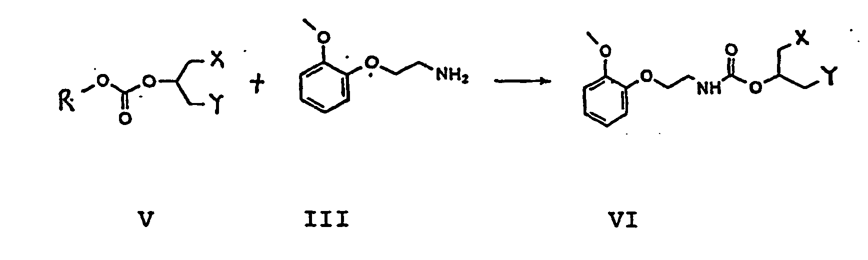

In addition to the desired product, the compound of formula (VI), also some [2-(2-(methoxy)-phenoxy)-ethyl]-carbamic acid-OR will be produced. The ratio of compound (VI) to [2- (2- (methoxy) -phenoxy) -ethyl]-carbamic acid-OR is determined by the leaving groups R-O and 1-X-3-Y-propan-2-oxy. If R=methyl or ethyl virtually no compound (VI) is formed, whereas if R=phenyl 90 % of the product of the reaction is the compound of the general formula (VI).

If R-O is more easily detached (that means forms a better leaving group) than the 1-X-3-Y-propan-2-oxy group the group R-O is released during said reaction in larger amount that 1-X-3-Y-propan-2-oxy and consequently the compound having the general formula (VI) is formed in higher amount than [2-(2-(methoxy)-phenoxy)-ethyl]-carbamic acid-OR.

According to the invention R is selected so that R-O is a better leaving group than 1-X-3-Y-propan-2-oxy. It is preferred that R is selected so the compound of the general formula (VI) comprises more than 75 % of the products of the above reaction, more preferred 90% or more.

For use according to the present invention examples of the group R includes: phenyl or phenyl substituted with one or more electrophilic substituents such as nitro, halogen or acetyl.

The person skilled in the art will appreciate that the term electrophilic should be understood in the usual meaning for example as defined in Morrison, R.T. and Boyd R.N.: Organic Chemistry, 4^{th} ed.(1983) Allyn and Bacon, Massachusetts, USA, page 326.

A phenyl group is a preferred group R.

The reaction is performed by mixing the compound of the general formula (V) and 2-(2-(methoxy-phenoxy)-ethylamine in a solvent that is inert during the conditions of the reaction, or without a solvent. Ethanol is a preferred solvent for the reaction because it is cheap, environmentally safe and further the isolation procedure is relatively easy when ethanol is used.

Next the compound (VI) is cyclisized for formation of the key compound of the general formula (VII):

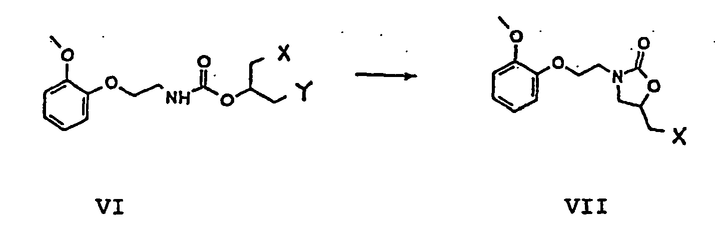

The reaction may be performed in an inert solvent in the presence of a base. The reaction is conveniently performed by addition of a solution of compound of the general formula (VI) to a suspension or solution of the base in the absence of oxygen. An example of a suitable solvent for the reaction is tetrahydrofuran (THF) in the presence of sodium hydride as the base. This reaction may be performed at a temperature from 0°C to the reflux temperature of 67°C.

It will be appreciated by the person skilled in the art that the result of the above reaction may be two different compounds depending on whether X or Y have been eliminated during the ring closure. Consequently if X is different from Y the product of the reaction will be a mixture of two compounds each represented by the general formula (VII), one compound where the X-group in the general formula (VII) is the X-group from compound (VI), and one compound where the X-group is the Y-group from compound (VI).

Further it will be appreciated that the ratio of molecules containing X to molecules containing Y will depend on how well the groups X and Y perform as leaving groups. If Y is easier to detach under the applied circumstances than X then the ratio will be high and vice versa.

As X and Y in the general formula (VI) are selected from the same definition of groups and because the groups will be lost during a subsequent step the notation of the general formula (VII) in reactions where it participates will for simplicity only be mentioned with an X even though it strictly spoken may comprise more than one compound.

Formation of the compound of the general formula (VII) may be achieved in other ways. For example it may be produced by reaction of 1-(2-isocyanato-ethoxy)-2-methoxy-benzene and epichlorohydrin. However this reaction has the disadvantage of using epichlorohydrin, which is not desired because of the risk conferred by this chemical. Further, the formation of 1-(2-isocyanato-ethoxy)-2-methoxy-benzene involves the use of phosgene in the usual synthesis, which is unacceptable from an environmental point of view.

Next the compound of the general formula (VII) is alkylated with 4-hydroxycarbazol for the formation of 5-(9H-carbazol-4-yloxymethyl)-3-[2-(2-methoxy-phenoxy)-ethyl]-oxazolidin-2-one (IX) :

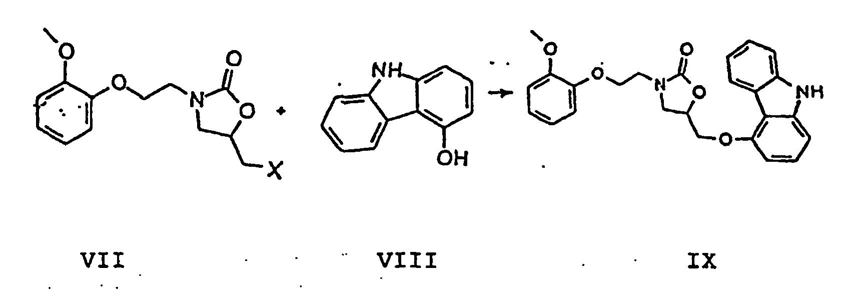

In principal the reaction may be performed in any solvent that is inert under the reaction conditions. However, surprisingly the selection of solvent greatly affects the reaction. In tetrahydrofuran the reaction hardly proceeds whereas the reaction performs well in alcohols having 1-6 carbon atoms.

A preferred solvent for the reaction is ethanol.

The reaction is most conveniently performed by refluxing a mixture of the reagents in a anhydrous alcohol such as absolute ethanol in the presence of a base such as potassium carbonate and a catalytic amount of potassium iodide.

The compound with the general formula (IX) may be purified in form of an acid addition salt, using methods well known to the person skilled in the art. The acid addition salt may in principle be an acid addition salt of any suitable acid. Examples of suitable acids for the formation of acid addition salts are: hydrochloric acid, hydrobromic acid, sulphuric acid, phosphoric acid, nitric acid, carbonic acid, acetic acid, butyric acid, succinic acid, malic acid, maleic acid, fumaric acid and citric acid.

As 4-hydroxycarbazol is very sensitive to oxygen and humidity in ambient air when warmed in solution the reaction is preferably performed under an inert atmosphere such as nitrogen or argon. Further it may be advantageous to add a small amount of an anti-oxidating agent such as potassium borohydride or sodium dithionite to improve the stability of 4-hydroxycarbazol under these conditions.

In the final step 5-(9H-carbazol-4-yloxymethyl)-3-[2-(2-methoxy-phenoxy)-ethyl]-oxazolidin-2-one (IX) or an acid addition salt thereof, is hydrolysed to 1-(9H-carbazol-4-yloxy)-3-[2-(2-methoxy-phenoxy)-ethylamino]-propan-2-ol, carvedilol, or an acid addition salt thereof:

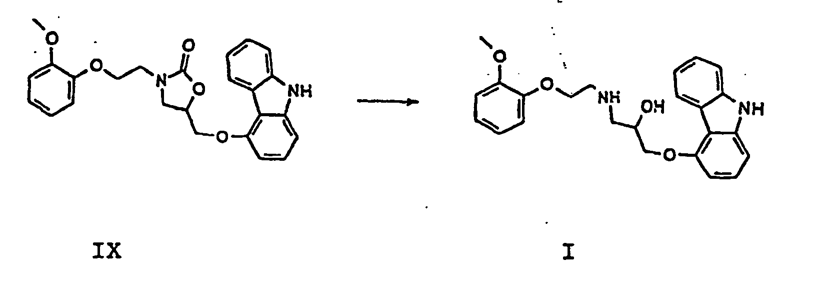

The hydrolysis may be performed by basic hydrolysis in an inert solvent in the presence of a base. A frequently used procedure for hydrolysis of ah oxazolidine of this type involves refluxing of the compound in a mixture of ethanol and aqueous base. Such a procedure is also applicable for this particular case.

The reaction is conveniently performed in 96% ethanol using solid sodium hydroxide as base.

Alternatively 5-(9H-carbazol-4-yloxymethyl)-3-[2-(2-methoxy-phenoxy)-ethyl]-oxazolidin-2-one (IX) may be hydrolysed to the desired compound, carvedilol, under acidic conditions for formation of an acid addition salt. The acid addition salt may in principle be an acid addition salt of any suitable acid. The examples of suitable acids given above in connection with the formation of an acid salt of the compound with the general formula (IX) apply likewise for the formation of an acid addition of carvedilol.

The formation of an acid addition salt of the carvedilol may be convenient in some protocols for the purification of said compound. Once obtained, an acid addition salt of carvedilol may be converted to the free base, carvedilol, using methods well known within the area.

The two final steps, the alkylation of the compound having the general formula (VII) with 4-hydroxycarbazol and the subsequent hydrolysis of the compound having the general formula (IX) may be performed without purification of the intermediate product (IX). This allows the reaction to be performed in one container, which would be advantageous because no purification or transfer of material is required which inevitably leads to loss and consumes time.

In order to perform these two final steps without intermediate purification a solvent should be selected in which both reactions perform well. An example of such a solvent is an alcohol having 1-6 carbon atoms, where ethanol is preferred.

Carvedilol contains one chiral carbon atom, the configuration of which is determined by the ring closure of the compound of the general formula (VI) to provide the compound of the general formula (VII) as shown above.

In one embodiment the starting reagent 1-X-3-Y-propan-2-ol is not a stereoisomer, i.e. the two groups X and Y are identical, in which case the reagent does not contain a chiral carbon atom, or the reagent is a racemic mixture; the resulting product compounds of the general formulas (VII), (IX) and carvedilol will be obtained as racemic mixtures containing approximately equal amounts of the two enantiomers.

Racemic mixtures of the compound of the general formulas (VII), (IX) or carvedilol may be resolved by methods known within the art.

In another embodiment the starting reagent 1-X-3-Y-propan-2-ol is a stereoisomer, which means that X is different from Y, and the reagent is obtained in a steroeisomeric pure form i.e. either in the R or the S enantiomer. The person skilled in the art will appreciate that as a result of the ring closure a mixture will be formed comprising a compound of the general formula (VII) in the R configuration containing exclusively either X or Y and a compound of the general formula (VII) in the S configuration containing exclusively the one of X and Y that is not contained in the R enantiomer. Thus the groups X and Y may serve as markers for the enantiomers.

In this embodiment X and Y may be selected so that one of the enantiomers is predominantly formed, by selection of X and Y such that one of these is a much better leaving group than the other. Alternatively X and Y may be selected to ease the separation of the R and S enantiomers of the compound of the general formula (VII) which enantiomers may be used to obtain carvedilol in the R and/or S configuration.

In a preferred embodiment X=Y=Z=chlorine and R=phenyl. The overall reaction scheme and the reactions for this embodiment are shown below. 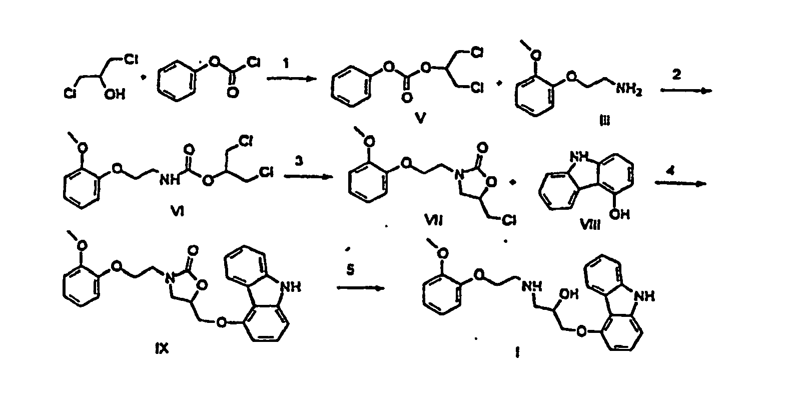
1. Formation of carbonic acid 2-chloro-1-chloromethyl-ethyl ester phenyl ester (V) from phenyl chloroformate and 1,3-dichloro-propan-2-ol.
2. Formation of [2-(2-methoxy-phenoxy)-ethyl]-carbamic acid 2-chloro-1-chloromethyl-ethyl ester (VI) by reaction of carbonic acid 2-chloro-1-chloromethyl-ethyl ester phenyl ester (V) and 2-(2-methoxy-phenoxy)-ethylamin (III).
3. Cyclisation of [2-(2-methoxy-phenoxy)-ethyl]-carbamic acid 2-chloro-1-chloromethyl-ethyl ester (VI) with sodium hydride to 5-chloromethyl-3-[2-(2-methoxy-phenoxy)-ethyl]-oxazolidin-2-one (VII) .
4. Alkylation of 5-chloromethyl-3-[2-(2-methoxy-phenoxy)-ethyl]-oxazolidin-2-one (VII) with 4-hydroxycarbazol (VIII) to 5-(9H-carbazol-4-yloxymethyl)-3-[2-(2-methoxy-phenoxy)-ethyl]-oxazolidin-2-one (IX).
5. Alkaline hydrolysis of 5-(9H-carbazol-4-yloxymethyl)-3-[2-(2-methoxy-phenoxy)-ethyl]-oxazolidin-2-one (IX) to 1-(9H-carbazol-4-yloxy)-3-[2-(2-methoxy-phenoxy)-ethylamino]-propan-2-ol (I), carvedilol.

The invention is now further illustrated by way of examples. The examples should be regarded as illusiratory and not construed as limiting in any way.

### EXAMPLES

### Example 1

### Formation of carbonic acid 2-chloro-1-chloromethylethyl ester phenyl ester (V)

To a mixture of 1,3-dichloropropan-2-ol (90.6 g, 0.70 mol) and triethylamine (71.1 g, 0.70 mol) in 400 ml toluene phenyl chloroformate (100 g, 0.64 mol) was added at a rate so that the temperature remained below 40°C. The reaction mixture was stirred for 3 hours and water (100 ml) was added. The phases were separated and the organic phase extracted twice with 1 M HCl (aq)(100 ml) and once with water (100 ml).
The toluene solution was concentrated under vacuum to afford the product.
Yield: 157.9 g (99%)
NMR:¹H(CDCl₃) 300 MHz δ=7.6-7.1 (5H,m), 5.2-5.0 (1H,m), 4.1-3.6(4H,m)
Bp=106.5-111°C at :0.1-0.25 mmHg
Elementary analysis: C:48.54 (48.22) H : 3.89 (4.05) HPLC (nucleosil C18, CH₃CN/H₂O 1:1, eluent:phosphate /triethylamine buffer pH 3, flow 0.9 ml/min) RT = 19.2 min

### Example 2

### Production of [2-(2-methoxy-phenoxy)-ethyl]-carbamic acid 2-chloro-1-chloromethyl-ethyl ester (VI):

To a suspension of 2-(2'-(methoxy)-phenoxy)-ethylamine hydrochloride (30.0 g, 0.15 mol) in 100 ml absolute ethanol triethylamine (14.9 g, 0.15 mol) was added. After stirring for 5 minutes carbonic acid 2-chloro-1-chloromethyl-ethyl ester phenyl ester (V) (36.7 g, 0.15 mol) was added and the reaction mixture was stirred overnight at room temperature. Water (50 ml) and toluene (100 ml) were added and the phases were separated. The organic phase was extracted twice with 2M HCl (aq) (25 ml) and four times with 2M NaOH (25 ml).
The toluene solution was concentrated under vacuum to afford the product.
Yield: 46.8 g (97%)
HPLC (nucleosil C18, CH₃CN/H₂O 1:1, eluent: phosphate / triethylamine buffer pH=3, flow 0.9 ml/min) RF=12.5 min, 90% ([2-(2-methoxy-phenoxy)-ethyl]-carbamic acid 2-chloro-1-chloromethyl-ethyl ester (VI)), RF=10.95 min, 10% ([2-(2-methoxy-phenoxy)-ethyl]-carbamic acid phenyl ester)
An analytical sample of the desired product [2-(2-methoxy-phenoxy)-ethyl]-carbamic acid 2-chloro-1-chloromethyl-ethyl ester was prepared by recrystallisation from ligroin (bp=80-100°C).
Mp = 64-66°C
NMR: ¹H (CDCl₃) 300 MHz δ = 7.26-6.82 (4H, m), 5.68 (1H, b), 5.15-5.08 (1H, m), 4.11-4.08 (2H, t, J=5Hz), 3.87 (3H, s), 3.77-3.68 (4H, m), 3.61-3.58 (2H, t, J=5Hz)
Elementary Analysis : C: 48.88 (48.46) H : 5.20 (5.32) N: 4.34 (4.35)

### Example 3

### Production of 5-chloromethyl-3-[2-(2-methoxy-phenoxy)-ethyl]-oxazolidin-2-one (VII):

[2-(2-methoxy-phenoxy)-ethyl]-carbamic acid 2-chloro-1-chloromethyl-ethyl ester (VI) (46.8 g, 0.15 mol) in THF (100 ml) was added to sodium hydride (7.8 g, 0.20 mol, 60% in mineral oil) suspended in THF (100 ml) under nitrogen at a rate so that the temperature did not exceed 67°C. The reaction mixture was stirred one hour at room temperature.
Next ligroin (bp=80-100°C) (50 ml) was added and water (100 ml) was carefully added to the mixture destroying excess of sodium hydride and dissolving the salts formed in the reaction.
The phases were separated and the organic phase was washed with water (50 ml).
The organic solution was concentrated under vacuum to afford the product.
Yield: 44.3 g (2.3 g mineral oil) 98%
An analytical sample was prepared by column chromatography on silica gel with ligroin (bp=80-100°C): ethyl acetate 1:1 as eluent.
NMR: ¹H (CDCl₃) 300 MHz δ = 7.00-6.87 (4H, m), 4.74-4.66 (1H, m), 4.19 (2H, t, J=5Hz), 4.09-3.61 (4H, m), 3.85 (3H, s), 3.68 (2H, t, J=5Hz)
Elementary Analysis : C: 54.67 (54.65) H : 5.64 (5.64) N: 4.86 (4.90)
MS: 285 (M+, 2%), 162 (100%)
HPLC (nucleosil C18, CH₃CN/H₂O 1:1, eluent: phosphate / triethylamine buffer pH=3, flow 0.9 ml/min) RT=7.3 min

### Example 4

### Production of 5-(9H-carbazol-4-yloxymethyl)-3-[2-(2-methoxy-phenoxy)-ethyl]-oxazolidin-2-one (IX):

A mixture of 5-chloromethyl-3-[2-(2-methoxy-phenoxy)-ethyl]-oxazolidin-2-one (VII) (35.6 g, 0.12 mol), 4-hydroxycarbazol (24.7 g, 0.13 mol), potassium carbonate (34.6 g, 0.25 mol), potassium iodide (0.2 g) and sodium dithionite (0.2 g) in absolute ethanol (150 ml) was heated to reflux under nitrogen atmosphere for 24 hours.
Ligroin (bp=80-100°C) (25 ml) was added followed by water (50 ml). The reaction mixture was stirred for 24 hours, and the product removed by filtration and dried under vacuum.
Yield: 39.5 g (76%)
HPLC (nucleosil C18, CH₃CN/H₂O 1:1, eluent: phosphate / triethylamine buffer pH=3, flow 0.9 ml/min) RF=17.7 min

### Example 5

### Production of 1-(9H-carbazol-4-yloxy)-3-[2-(2-methoxy-phenoxy)-ethylamino]-propan-2-ol, carvedilol (I):

5-(9H-carbazol-4-yloxymethyl)-3-[2-(2-methoxy-phenoxy)-ethyl]-oxazolidin-2-one (IX) (10.0 g, 23.2 mmol) was refluxed in 96% ethanol (100 ml) containing sodium hydroxide (4.0 g, 0.1 mol) under a nitrogen atmosphere. After cooling to room temperature, a 1:1 mixture of toluene and THF (50 ml) followed by water (100 ml) were added to the reaction mixture.
The phases were separated and the water phase extracted three times with a 1:1 mixture of toluene and THF (50 ml)
The combined organic phase was dried, and concentrated under vacuum to afford the product as an oil.
The product was recrystallised from ethylacetate (25 ml).
Yield: 6.6 g (70%)
M.p: 114-116 °C
Elementary Analysis: C: 70.55 (70.92) H : 6.21 (6.45) N: 6.80 (6.89)
HPLC (nucleosil C18, CH₃CN/H₂O 1:1, eluent: phosphate / triethylamine buffer pH=3, flow 0.9 ml/min) RT=4.8 min

## Claims

1. A compound having the general formula (V) 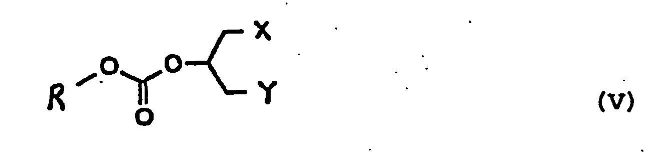 wherein X and Y independently are selected from: fluorine, chlorine, bromine, iodine, besylates, tosylate, mesylate, brosylates, nosylates, triflates, nonaflates or tresylates; and
R is selected from: phenyl and phenyl substituted with one or more substituents independently selected from: fluorine, chlorine, bromine, iodine, nitro and acetyl groups;
with the proviso that the compound having the general formula (V) cannot be 2-chloro-1-(chloromethyl)ethyl pentachlorophenyl carbonate or 2-bromo-1-(bromomethyl)ethyl pentachlorophenyl carbonate.

2. A compound according to claim 1, **characterized in that** X=Y=chlorine and R=phenyl.

3. A process for the manufacture of a compound having the general formula (V), charac-terized in reacting 1-X-3-Y-propan-2-ol with R-Z-formate, 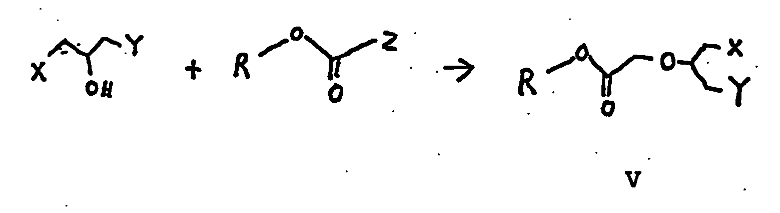 wherein X,Y and Z each are leaving groups; and
R is selected from: phenyl and phenyl substituted with one or more electrophilic substituents.

4. A process according to claim 3, wherein X and Y independently are selected from: fluorine, chlorine, bromine, iodine, besylates, tosylate, mesylate, brosylates, nosylates, triflates, nonaflates or tresylates; and Z is selected from: fluorine, chlorine, bromine and iodine.

5. A process according to claims 3-4, **characterized in that**, R is selected from: phenyl and phenyl substituted with one or more substituents independently selected from: fluorine, chlorine, bromine, iodine, nitro and acetyl groups.

6. A process according to claims 3-5, **characterized in that** X=Y=Z=chlorine and R=phenyl.

7. Use of the compound having the formula (V) for the preparation of the compound having the formula (VI).

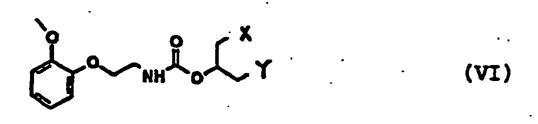

8. Use of the compound having the formula (V) for the preparation of the compound having the formula (VII).

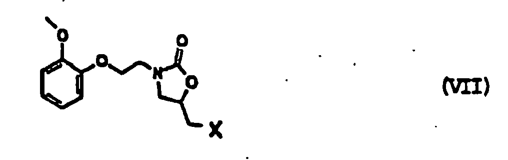

9. Use of the compound having the formula (V) for the preparation of carvedilol.

## Patentansprüche

1. Verbindung der allgemeinen Formel (V) 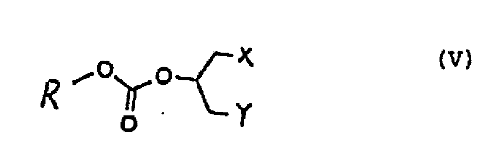 wobei X und Y unabhängig unter: Fluor, Chlor, Brom, Jod, Besylaten, Tosylat, Mesylat, Brosylaten, Nosylaten, Triflaten, Nonaflaten oder Tresylaten ausgewählt sind; und
R ausgewählt ist unter: Phenyl und Phenyl, das durch einen oder mehrere Substituenten substituiert ist, die unabhängig ausgewählt sind unter: Fluor, Chlor, Brom, Jod, Nitro und Acetylgruppen;
unter der Bedingung, dass die Verbindung der allgemeinen Formel (V) nicht 2-Chlor-1-(chlormethyl)ethyl Pentachlorphenylcarbonat oder 2-Brom-1-(brommethyl)ethyl Pentachlorphenylcarbonat sein kann.

2. Verbindung nach Anspruch 1, **dadurch gekennzeichnet, dass** X=Y=Chlor und R=Phenyl ist.

3. Verfahren zur Herstellung einer Verbindung der allgemeinen Formel (V), **gekennzeichnet durch** die Reaktion von 1-X-3-Y-Propan-2-ol mit R-Z-Formiat, 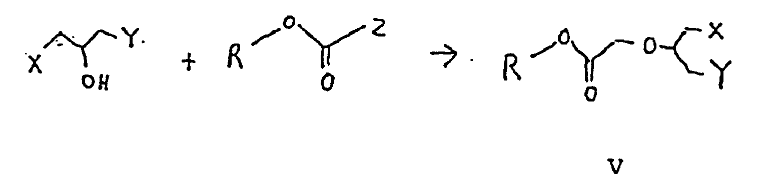 wobei X,Y und Z jeweils Abgangsgruppen sind; und
R ausgewählt ist unter: Phenyl und **durch** einen oder mehrere elektrophile Substituenten substituiertem Phenyl.

4. Verfahren nach Anspruch 3, wobei X und Y unabhänig ausgewählt sind unter: Fluor, Chlor, Brom, Jod, Besylaten, Tosylat, Mesylat, Brosylaten, Nosylaten, Triflaten, Nonaflaten oder Tresylaten; und Z ausgewählt ist unter: Fluor, Chlor, Brom und Jod.

5. Verfahren nach Anspruch 3 bis 4, **dadurch gekennzeichnet, dass** R ausgewählt ist unter: Phenyl und Phenyl, das durch einen oder mehrere Substituenten substituiert ist, die unabhängig ausgewählt sind unter: Fluor, Chlor, Brom, Jod, Nitro und Acetylgruppen.

6. Verfahren nach Anspruch 3 bis 5, **dadurch gekennzeichnet, dass** X=Y=Chlor und R=Phenyl ist.

7. Verwendung der Verbindung der Formel (V) zur Herstellung der Verbindung der Formel (VI).

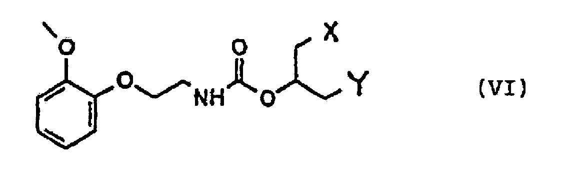

8. Verwendung der Verbindung der Formel (V) zur Herstellung der Verbindung der Formel (VII).

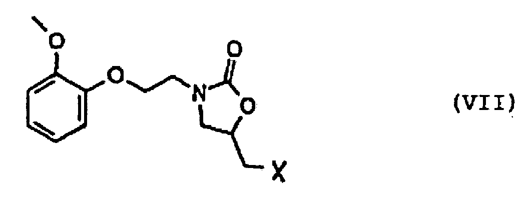

9. Verwendung der Verbindung der Formel (V) zur Herstellung von Carvedilol.

## Revendications

1. Composé ayant la formule générale (V) 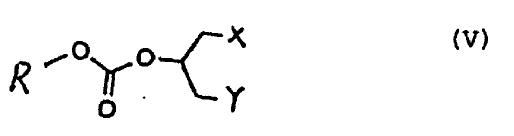 dans laquelle X et Y sont indépendamment choisis parmi: fluor, chlore, brome, iode, besylates, tosylate, mesylate, brosylates, nosylates, triflates, nonaflates ou tresylates; et
R est choisi parmi: phényle et phényle substitué par l'un ou plusieurs substituants indépendamment choisis parmi: fluor, chlore, brome, iode, groupes nitro et acétyle;
avec la condition que le composé ayant la formule générale (V) ne peut pas être 2-chloro-1-(chlorométhyle)éthyle pentachlorophenyl carbonate ou 2-bromo-1-(bromométhyl)éthyle pentachlorophenyl carbonate.

2. Procédé selon la revendication 1, **caractérisé en** X=Y=chlore et R= phényle.

3. Procédé destiné à la préparation d'un composé ayant la formule générale (V), **caractérisé en** réagissant 1-X-3-Y-propane-2-ol avec R-Z-formiate, 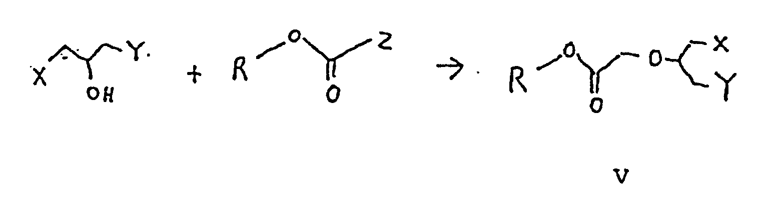 dans laquelle X, Y et Z sont des groupes de sortie; et
R est choisi parmi: phényle et phényle substitué par un ou plusieurs substituants électrophiles.

4. Procédé selon la revendication 3, dans lequel X et Y sont indépendamment choisis parmi: fluor, chlore, brome, iode, besylates, tosylate, mesylate, brosylates, nosylates; et Z est choisi parmi: fluor, chlore, brome et iode.

5. Procédé selon les revendications 3-4, **caractérisé en ce que** R est choisi parmi: phényle et phényle substitué par l'un ou plusieurs substituants indépendamment choisis parmi: fluor, chlore, brome, iode, groupes nitro et acétyle.

6. Procédé selon les revendications 3-5, **caractérisé en** X=Y=chlore et R= phényle.

7. Utilisation du composé ayant la formule (V) pour la préparation du composé ayant la formule (VI).

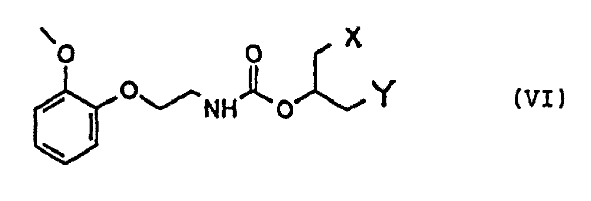

8. Utilisation du composé ayant la formule (V) pour la préparation du composé ayant la formule (VII).

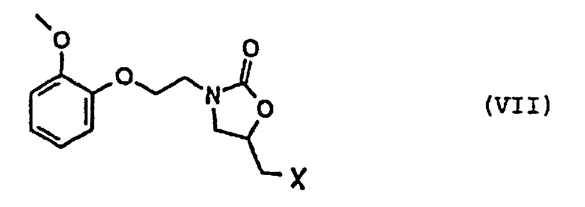

9. Utilisation du composé ayant la formule (V) pour la préparation de carvedilol.
